# EUROPEAN PATENT SPECIFICATION

(11) **EP 1 984 061 B1**
(45) Date of publication and mention of the grant of the patent: **25.01.2012**
(21) Application number: 07750421.5
(22) Date of filing: 09.02.2007
(51) Int. Cl.: A61M 29/00, A61M 31/00

(54) **Coaxial PTA balloon**
Koaxialer PTA-Ballon
Ballon pour ATP coaxial

(30) Priority: 14.02.2006 US 773243 P
(43) Date of publication of application: 29.10.2008
(73) Proprietor: C.R.Bard, Inc., Murray Hill, NJ 07974 (US)
(72) Inventor: ALMAZAN, Daniel, Avondale, AZ 85323 (US)
(74) Representative: HOFFMANN EITLE
(86) International application number: PCT/US2007/003584
(87) International publication number: WO 2007/095125

(56) References cited:
- WO-A1-81/02110
- US-A- 4 422 447
- US-A- 5 246 421
- US-A- 5 330 429
- US-A- 5 676 654
- US-A- 6 142 993
- US-A1- 2003 158 516

## Description

### PRIORITY

This application claims the benefit of priority to U.S. Provisional Patent Application No. 60/773,243, filed February 14, 2006, to which the reader is referred.

### BACKGROUND

Catheters are used as essential medical instruments for diagnosis and treatment of vascular diseases. In recent years, specially designed catheters used in combination with guide wires have allowed physicians to perform minimally invasive procedures to treat ailments intravascularly. This is typically done by inserting a catheter into the interior of a body vessel, using a guide wire to advance the catheter along the vessel to reach the point of interest.

Balloon catheters (e.g., catheters with distally located balloons) have been used to dilate vessels and clear obstructions within the vessels. For example, percutaneous transluminal angioplasty (PTA) balloons may be used to temporarily occlude or dilate vessels, and may be used in the treatment of various vascular diseases such as aneurysms, arteriovenous malformations and arteriovenous fistulas, where the control of blood flow during treatment is typically necessary. Balloon catheters may also include lumens for delivery of fluids or therapeutics. Balloon catheters have also been used to help position and deploy medical devices such as stents and occlusive coils (e.g., vasooclusive coils). For example, stents deployed by expanding a balloon catheter may be used to treat arterial stenosis secondary to atherosclerosis.
US 2003/0158516 A1 and US Patent 5,330,429 each disclose a balloon catheter falling within the scope of the precharacterising portion of Claim 1 below. US Patent 5,676,654 also discloses a balloon catheter relevant to the subject-matter of the present application.

In many applications, balloon catheters are inserted into a subject's vasculature (including insertion of the balloon catheter within a guide catheter or sheath). Once inserted, the balloon may be expanded for therapeutic or treatment purposes, and later deflated, and withdrawn. Thus, the diameter (e.g., cross-sectional size) of a catheter in the un-inflated state may be critical to accessing narrow or difficult to reach regions of a body. Furthermore, it may be desirable to inflate a balloon catheter from a narrow profile into a broad profile, and then completely and predictably collapse the catheter back into a low-profile un-inflated state. The following references are related to balloon catheters: USPN 6,135,982, USPN 5,919,163, USPN 6,764,441 and US Publication No. 2005/0055077.

Most balloon catheter designs generally include one or more balloon that is attached near the distal end of the catheter. When the balloon is in the un-inflated state, the collapsed balloon is typically folded or gathered around the distal end of the catheter. Often the un-inflated balloon folds over the distal tip of the catheter. Such designs result in a raised profile for the catheter around the region of the balloon, and also a substantial increase in the diameter of the catheter. This increase in diameter significantly limits where the catheter may be deployed in the vascular system. Vessels distal from the aorta may become hard to reach as the diameter of the catheter is increased. Furthermore, securing the balloon on the surface of the catheter may require clamps, bonding materials, or other devices attached to the surface of the catheter, tending to cause an uneven profile on the surface of the catheter, which may make advancing the catheter in narrow vessels even more difficult and potentially dangerous. Implementation of a balloon along the shaft of the catheter also tends to significantly limit the flexibility of the catheter around the balloon section, and make the catheter difficult to maneuver.

In addition, the size of the balloon (e.g., the length and width) in the inflated state is not generally controllable in most commercially available balloon catheters. Most balloon catheters inflate to the same size (e.g., length) and overall shape. However, there are many instances when it would be beneficial to control the final shape and/or size that the balloon portion of a balloon catheter in the expanded state. For example, when performing minimally-invasive surgical techniques in vessels of different lengths and diameters using a single balloon catheter.

Therefore, applicant has recognized that a balloon catheter with a low profile when un-inflated, or during deflation, and which may be size-adjustable and highly maneuverable is much desired and could be of significant medical value.

### BRIEF SUMMARY OF THE INVENTION

Described herein are balloon catheters (including PTA balloon catheters), catheter assemblies, and methods of using these catheters. In general, these balloon catheters include an inflatable member forming an inflatable region at the distal end of the balloon catheter. At least one end of the inflatable member is attached to the distal end of the catheter body, and the other end is attached to a movable member that is slidably disposed about the catheter body. The inflatable region can be inflated by applying fluid (e.g., air, saline, etc.). Inflating the inflatable region may cause the movable member to move toward the end of the inflatable member that is attached to the catheter body.

In one embodiment, a balloon catheter includes a catheter body having an inflation lumen and an opening in a wall at the distal end of the catheter body that is in fluid communication with the inflation lumen and an inflatable member. The inflatable member has a first end and a second end. The second end is fixed to a distal end of the catheter body, and the first end is coupled to a movable member that is disposed about the catheter body and forms a seal with the catheter body. The balloon catheter also comprises an outer delivery sheath with a lumen. The delivery sheath has an opening at the distal end that is configured to permit passage of the catheter body and the inflatable member, but prevents the passage of the movable member. For example, the movable member may be located within the delivery sheath, and allow it to slide axially along the balloon catheter. The delivery sheath may limit the movable member by preventing it from sliding distally through the opening in the delivery sheath. For example, the opening in the delivery sheath at the distal end may be smaller than the diameter of the moveable member, or it may be coupled to the moveable member.

In general, the movable member moves axially along the elongated length of the catheter body. As mentioned, this motion may be limited (e.g., by a delivery sheath or other mechanism). For example, the balloon catheter may include a shoulder that is located adjacent to the opening in the distal end wall of the catheter body. This shoulder may be configured to limit movement of the movable member in a distal direction. Thus, the shoulder may stop movement of the moveable member in the distal direction (e.g., past the opening in the distal end wall).

The moveable member also forms a seal with the catheter body that substantially prevents leakage of fluid from the inflatable region. "Substantially preventing leakage" of fluid may mean that all, the majority, or only some of the fluid is prevented from leaking out of the inflatable region through the moveable member. In some embodiments, the moveable member includes a gasket or lip to prevent substantial amounts of fluid from leaking from the inflatable region. As mentioned, some fluid may pass from the inflatable region through the movable member. The moveable member may be configured to limit or direct the passage of fluid from the inflatable region. For example, fluid may pass into a channel within an outer delivery sheath.

The movable member may be configured as an annulus (e.g., as a cylindrical annulus) having threads that are formed on the inner cylindrical surface. The threads may mate with corresponding threads formed on the catheter body, so that the movable member translates and rotates as the inflatable member is inflated.

The inflatable member may be a non-compliant balloon whose inner surface area remains essentially constant in an inflated or un-inflated state. For example, the balloon may be made of a substantially non-compliant material, or the balloon may include a non-compliant material such as a mesh (e.g., a metal mesh) encasing at least part of the balloon. In some embodiments, the balloon is a compliant balloon made from thermoplastic polymers. In some embodiments, the balloon catheter includes a guidewire lumen that is coupled to the tip of the catheter. Thus, the distal tip may also have a lumen configured for passage of the guidewire.

The balloon catheter may also include a tip that is connected to the distal end of the catheter body. The tip is typically an atraumatic tip, or has an atraumatic configuration. An atraumatic tip may prevent undesirable puncture and damage to tissue. In some embodiments, the distal end of the inflatable member is coupled to a locking member that is permanently attached to the catheter body.

In the embodiment, the catheter includes an outer delivery sheath and a balloon catheter. The delivery sheath that has a sheath lumen and a distal end opening. The balloon catheter may be positioned at least partly within the lumen of the delivery sheath. The balloon catheter typically includes a catheter body with an inflation lumen, and an opening in a distal end wall that is in fluid communication with the inflation lumen. The inflatable member may have a distal end that is fixed to a distal end of the catheter body and a proximal end that is coupled to a movable member. The movable-member may be in fluid-tight engagement with an outer surface of the catheter body. The moveable member may substantially prevent leakage from within the inflatable region, as described above.

The opening at the distal end of the sheath body may have a diameter that is smaller than a diameter of the movable member. Thus, the movable member may move over the opening so that the opening is completely covered underneath the movable member when the movable member is positioned over the top of the opening. As described above, the movement of the movable member (particularly relative to the opening at the distal end) may be limited. For example, an inner surface of the delivery catheter may have a shoulder that is adjacent to the opening at the distal end, and the shoulder may be configured to stop the movable member from moving past it in the distal direction. The balloon catheter may also include a tip that is attached to the distal end of the catheter body. The tip may include a lumen (e.g., configured for passage of a guidewire). This lumen through the tip may be continuous with a guidewire lumen extending through the catheter body.

The movable member may include an annulus with threads formed on the inner cylindrical surface that mate with corresponding threads or channels formed on the catheter body. Movement of the movable member results in translation and rotation of the movable member as the inflatable member is inflated or deflated.

Also described herein are methods of deploying a balloon catheter. These methods may include introducing a delivery catheter having a lumen and a distal end opening into a blood vessel or body cavity, moving a balloon catheters through the delivery catheter lumen toward a distal end of the delivery catheter (the balloon catheter typically includes a catheter body and an inflatable member that are coupled to a movable member near the distal end of the balloon catheter), advancing the distal end of the catheter body through the distal end opening of the delivery catheter, and inflating the inflatable member to move the movable member with respect to the outer surface of the catheter body.

The opening at the distal end of the delivery catheter may be configured to prevent passage of the movable member. Inflating may involve the movable member contacting an inner surface of the delivery catheter adjacent the distal end opening. Inflating may mean delivering air or fluid at a pressure in the range of about 5 atm to about 20 atm through an inflation lumen of the catheter body so that it exits an opening in a wall of the catheter body that is positioned between the distal end of the catheter body and the movable member.

The step of moving a balloon catheter through the delivery catheter lumen toward a distal end of the delivery catheter may mean rotating and translating the movable member to unwind the inflatable member. Rotating and translating may involve restricting the maximum outer diameter of the inflatable member as a function of the location of the movable member relative to the distal end of the catheter body.
Accordingly, the present invention provides a balloon catheter as recited in Claim 1 below. Dependent claims therefrom are directed to particular preferred embodiments thereof.

These and other embodiments, optional features and advantages will become more apparent to those skilled in the art when taken with reference to the following more detailed description of the invention in conjunction with the accompanying drawings that are first briefly described.

### BRIEF DESCRIPTION OF THE DRAWINGS

The accompanying drawings, which are incorporated herein and constitute part of this specification, illustrate presently preferred embodiments of the invention together with illustrative examples related thereto, and, together with the general description given above and the detailed description given below, serve to explain features of the invention.

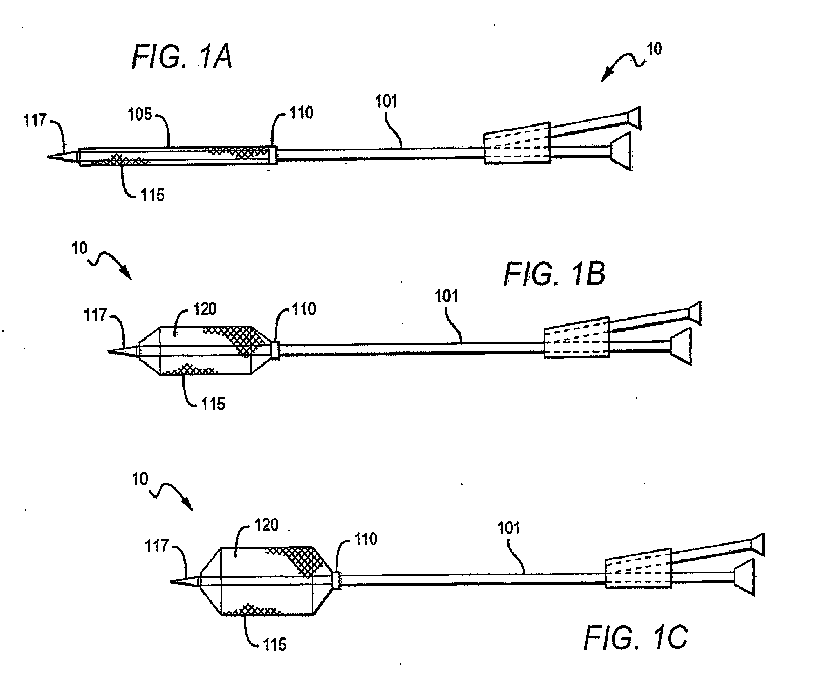

FIG. 1A illustrates one variation of a balloon catheter as described herein in an un-inflated state.

FIG. 1B shows the balloon catheter of Fig. 1A in a partially inflated state.

FIG. IC shows the balloon catheter of Fig. 1A in an inflated state.

FIG. 2A shows a perspective view of a movable member.

FIG. 2B shows a cross-sectional view of a movable member coupled by a screw-like threading to a catheter body.

FIG. 2C shows a cross-sectional view of a movable member.

FIG. 2D shows a cross-sectional view of a movable member forming a seal with the catheter body.

FIG. 3A shows a cross-section of a movable member coupled to a positioner.

FIG. 3B shows a cross-section of the distal region of a balloon catheter including a shoulder.

FIG. 3C shows a cross-section of the distal region of a balloon catheter.

FIG. 4A shows another variation of a balloon catheter as described herein, in an un-inflated state.

FIG. 4B shows the balloon catheter of FIG. 2A in an inflated state.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. Hence, the invention is not limited to the preferred embodiments described exemplarily herein. Moreover, this description will clearly enable one skilled in the art to make and use the invention, and describes several embodiments, adaptations, variations, alternatives and uses of the invention, including what is presently believed by applicant to be the best mode of carrying out the invention.

As used herein, the terms "about" or "approximately" for any numerical values or ranges indicate a suitable dimensional tolerance that allows the part or collection of components to function for its intended purpose as described herein. Also, as used herein, the terms "patient", "host" and "subject" refer to any human or animal subject and are not intended to limit the systems or methods to human use, although use of the subject invention in a human patient represents a preferred embodiment.

Described herein are balloon catheters, catheter assemblies including balloon catheters, and methods of using balloon catheters. While the examples provided herein are directed toward PTA (or percutaneous transluminal angioplasty) balloon catheters, these catheters may be configured for any appropriate use, including non-angioplasty uses. For example, the balloon catheters, catheter assemblies, and methods of use may be used to deliver one or more medical devices (e.g., stents) within a vessel or other body cavity, or to occlude blood flow as part of a medical or therapeutic procedure.

In general, the catheters described herein are elongate, flexible tubes configured for insertion into a body cavity. These catheters may include one or more lumens extending at least part of the length of the catheter, which may carry fluid or provide a passage for other devices or instruments, including additional catheters. The term "balloon" may refer to any appropriate device which is expandable from a collapsed (e.g., un-inflated) configuration to an expanded (e.g., inflated) configuration.

### Balloon Catheters

The balloon catheters described herein include a catheter body, an inflatable member (e.g., a balloon), and a movable member. One end of the inflatable member is connected to the catheter body, and another end of the inflatable member is connected to the movable member. This arrangement of catheter body, inflatable member and movable member allows the inflatable member to have a very low profile when un-inflated, and may allow more precise control over the size of the inflatable member when it is inflated. The inflatable member portion of the balloon catheter may also be described as a coaxial inflatable member because it coaxially surrounds the catheter body in both the inflated and un-inflated states.

Figs 1A and 1B illustrate one variation of a balloon catheter 10 and show the relationship of the catheter body 101, movable member 110, and inflatable member 105, as well as additional features that may be part of these balloon catheters. In Fig. 1A, a first end of the inflatable member 105 is coupled to the movable member 110, and a second end of the inflatable member 105 is fixed to the catheter body 101. The second end of the inflatable member is coupled to the catheter body 101 near the distal end of the catheter body. The second end of the inflatable member may be fixed to the catheter body in any appropriate manner that secures the inflatable member to the catheter body even when the inflatable member is inflated (e.g., under pressure). For example, the second end of the inflatable member may be clamped, glued, fastened, etc. In Figs. 1A and 1B the second end of the inflatable member is secured near the distal end of the catheter body immediately before a distal tip of the catheter body 117. In some variations, the second end (the distal end) of the inflatable member is attached to the extreme distal end of the catheter body (and may be configured to extend beyond the distal end of the catheter body). In other variations, the second end of the inflatable member is attached more proximally to the catheter body. The second end of the inflatable member may be secured to the distal region of the catheter body by a locking member that is permanently attached to the catheter body.

The catheter body 101 is typically a flexible elongated member having a distal end and a proximal end. The catheter body may also include an outer surface and may include at least one inflation lumen extending axially (e.g., along the length of the catheter body) for applying or removing fluid to inflate the inflatable region formed between the first and second ends of the inflatable member. The catheter body may also include additional passageways or lumen. For example, the catheter body may include a guidewire lumen, or a lumen for passing a medical instrument (e.g., an electrode, a visualization device, etc.). The passage(s) or lumen of the catheter body typically passes from the proximal end of the catheter body to the distal end. The inflation lumen may be in open fluid communication with one or more openings in the distal end wall of the catheter body, and is open to at least one inflation area (inflatable region). The inflation area may be the region between the inflatable member (e.g., balloon) and the outer region of the catheter body, as shown in more detail below. It should be noted that more than one opening into the inflation lumen may be used inflate a balloon.

The catheter body may be made of any of a wide variety of materials that are suitable for a catheter. That is to say, the catheter body may be made of metallic alloys, metals, polymers, or may be an assemblage or composite of such materials. For instance, suitable alloys include the group known as superelastic alloys, appropriate stainless steels, various engineering polymers optionally containing fibrous reinforcing materials, woven or wound assemblages of these materials, and others that are generally sufficient strength, flexibility and, optionally, be substantially non-kinking in such service. Examples of suitable superelastic alloys include nickel titanium alloys (e.g., 48-58 atomic % nickel and optionally containing modest amounts of iron); copper/zinc alloys (38-42 weight % zinc); copper/zinc alloys containing 1-10 weight % of beryllium, silicon, tin, aluminum, or gallium; or nickel/aluminum alloys (36-38 atomic % aluminum). Widely used NiTi alloys, generally known as "Nitinol," are described in USPN 3,174,851, USPN 3,351,463, and USPN 3,753,700. Such alloys tolerate significant flexing even when drawn as a very small diameter wire. The formation of medical devices from Nitinol alloys having both superelastic and shape memory properties is well known in the art and described in USPN 4,795,458, USPN 5,037,427, and WO 94/16629, to each of which the reader is referred. Other superelastic materials such as those described by Saito, et al. in SCIENCE, 300, 464-467 (2003) of titanium, zirconium, vanadium, niobium, and tantalum together with a small amount of oxygen, may also to be appropriate materials. Anti-kinking facilities may be enhanced by wrapping a tubing of such a material with, e.g., a braided or coiled exterior layer. In variations of the system where the catheter body is multi-lumened, the catheter body may most easily be formed had via polymer extrusion. Various polyimides are suitable as relatively strong but stiff materials for the shaft of the catheter body.

Different regions of the catheter body (e.g., different axial regions) may have different structures or different properties, and may be made of different materials. In some variations, a higher level of torqueability or stiffness may be provided at the proximal end of the catheter body compared to the distal end, particularly when the body is polymeric. In such instances, some portion of the proximal section of the catheter body may be formed using metallic tubing to reinforce the body, e.g., by placement of the metallic tubing outside and perhaps glued or otherwise sealed to the inner portion. The use of various braids or coils wrapped or otherwise situated around the catheter body to reinforce the more proximal section of the catheter body may be useful. The catheter body may be initially formed, e.g., by co-extrusion with a braid or coil placed interior to the body wall for at least a portion of the body length. The catheter body may be of a constant diameter, or it may be tapered with the smaller end of the taper toward the distal end of the body. As described above, the catheter body is typically attached to an inflatable member, as shown in the figures.

The inflatable member may include any appropriate material. For example, the inflatable member may be a compliant balloon, a semi-compliant balloon, or a non-compliant balloon. A non-compliant balloon is generally a balloon whose overall surface area (e.g., the inner surface area) remains essentially constant in an inflated and un-inflated states. A compliant balloon may include a material whose surface area may change between the inflated and un-inflated states. The meaning of "complaint," "semi-compliant" and "non-compliant" when referring to the balloons and materials making up the balloons are not strict. For example, non compliant balloons may have some measure of compliance with a lumen, once expanded. Furthermore, compliant balloons including certain types of elastic material may reach a point upon expansion where they are no longer capable of compliance with an exterior force, and compliant balloons may not shrink to their previous shape after hyper-inflation. Nevertheless, there are approximate understandings in the medical arts relating to such terminology.

The materials used in forming the various balloons suitable for the described device may be characterized as elastic, elastomeric, non-elastic, as are currently known and used in the field of polymer engineering. The inflatable members described herein may be made of any of the materials otherwise found in medical balloon devices used in medical treatments. Examples of materials useful in making compliant (or elastic) balloons include various polymeric materials (including materials already known to be useful for making compliant medical balloons), e.g., elastomeric membranes having a high degree of linearity (non-plasticity) for a wide range of stress and strain values. Such materials include various Silicones, latex, Kraton, various thermoplastic elastomers (TPE's) particularly styrene-ethylene/butylene-styrene block copolymers (SEBS)-based TPE's (such as C-Flex), polysiloxane modified SEBS and their associated families, polyvinylchloride (PVC), crosslinked polyolefins such as polyethylene, and various polyurethanes. Examples of materials used in making noncompliant balloons (e.g., inelastic balloons) include many of the polyamides (e.g., the Nylons), thermoplastic polyamides, polyesters, polyphenylene sulfides, ultra-high molecular weight polyethylene, and polyethyleneterephthalate (PET). PET is especially interesting due to its capacity for easy production of very thin wall balloons.

The balloon material may be selected or treated to allow the chosen inflation fluid to permeate through the balloon wall. The treatment may be chemical or physical. This ability may be useful when, for instance, the fluid is used to treat a medical problem on the bodily structure to which the balloon is applied. The polymeric material making up the balloons (and other components and sub-assemblies of the system) may further include one or more solid radio-opaque materials such as particles of tantalum, gold, tungsten, platinum, tantalum oxide, barium sulfate, and their mixtures when the designer sees the need for an amount of radio-opacity. Such materials may be particularly useful in tracking or otherwise visualizing the inflatable member or other components of the device.

In general, the inflatable member (e.g., balloons) used in the described devices include balloons that expand when a fluid in imposed on the interior of that balloon (e.g., under pressure). As described, an inflatable member may be either a compliant or non-compliant balloon (or a semi-compliant balloon). The choice of compliant and non-compliant materials may be based on the application, or the configuration of the balloon catheter. For example, non-compliant balloons may be capable of accepting very high inflation pressures, and may present a stiff or outer face that may be useful (e.g., for treating calcified plaque on an arteries), particularly when one wants to exert very high pressures without increasing the size of the balloon beyond a pre-specified diameter. Further, it is generally believed that balloons made of elastic materials may be advantages over non-elastic materials, because elastic materials (e.g., rubbers) may return to their original profile after deflating. Noncompliant balloons may simply fold after deflation. As discussed further below, the balloon catheters described herein may minimize the profile of both non-compliant balloons and compliant balloons.

A non-compliant balloon may also be made of an elastic material. For example, in Figs. 1A-1C, the balloon shown includes a mesh or covering 115 that expands as the balloon inflates. The mesh may be integral (e.g., part of) the material forming the balloon, or it may be on top of the balloon material (e.g., the mesh may be glued or otherwise adherent to the balloon material. The mesh 115 may be a forming member that is configured to shape the balloon in either (or both) the inflated or un-inflated states. In some variations, the mesh may provide a compression force on the balloon. For example, the force of the mesh may help compress the balloon from the expanded state shown in Fig. 1C into the unexpanded state shown in Fig. 1A. Combinations of compliant and non-compliant materials may be used. In some variations, a combination of such materials (e.g., elastic and in-elastic materials or regions of material) may be used to shape the inflatable member in either the inflated or un-inflated states.

Returning to Fig. 1A, the first end of the inflatable member 105 shown is attached to a movable member 110. The movable member 110 is shown as an annulus that encircles the catheter body, and is axially movable (e.g., slideable) along the catheter body in the proximal and distal directions. In some variations, the movable member is also radially movable (e.g., can be rotated around the axial body). The first end of the inflatable member may be coupled to the movable member in any appropriate fashion. For example, the first end of the inflatable member may be secured to the movable member by a clamp, by an adhesive, by a fastener, etc. The first end of the inflatable member (e.g., the balloon) is generally coupled completely around the perimeter of the movable member. Similarly, the second end of the inflatable member is generally coupled completely around the catheter body.

The connection of the inflatable member to the catheter body at one end, and to the movable member at the other end, generally forms an inflatable region 120 between the outer surface of the catheter body 101 and the inner surface of the inflatable member 105. The inflatable region may expand radially outward as the inflatable member is inflated and as the movable member is moved distally, as shown in Fig. 1B (showing a partially inflated member) and Fig. 1 C (showing a fully inflated member). The inflatable member is shown as a tube-like structure with the distal and proximal ends attached as described above. For example, in some variations, the inflatable member may include multiple layers. The layers may form separate (e.g., separately inflatable) regions. In some variations, the inflatable region 120 is bounded by the catheter body 101. In other variations, the inflatable region 120 is completely bounded by an inflatable member (e.g., balloon).

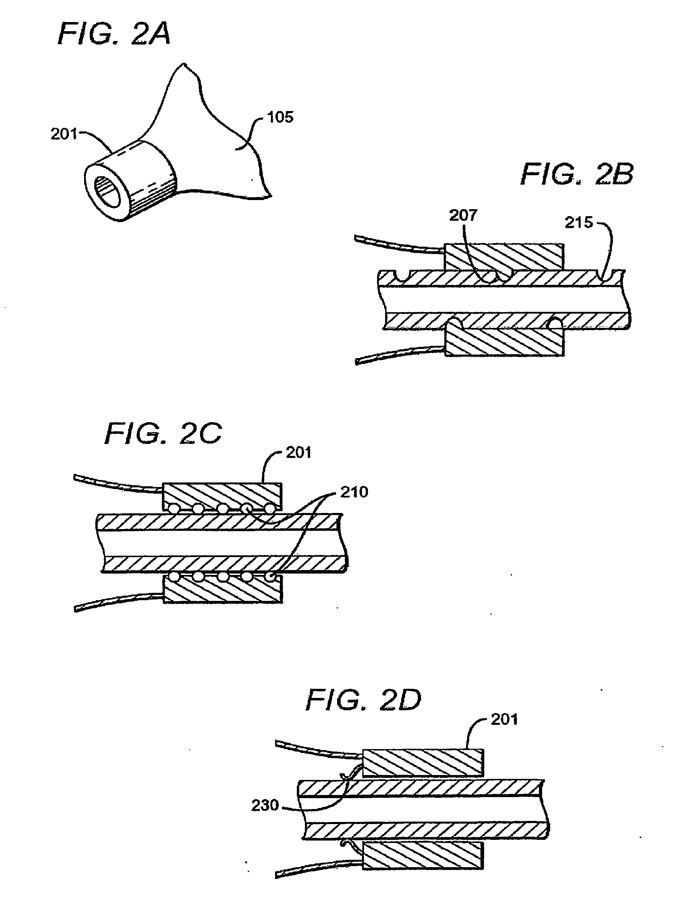

The moveable member may be any member that may connect to the inflatable member and may slide along the catheter body. Figs. 2A-2D illustrate examples of movable members usable in the present invention. Fig. 2A shows a perspective view of a movable member 201 configured as an annulus. The inflatable member 105 is attached to the distal region of the movable member 201. The inflatable member may be attached to any portion of the movable member, including the middle region or the proximal region (which may result in at least a portion of the movable member being within the inflatable region).

The inner surface of the movable member may be configured to move along the catheter body. For example, the inner surface may engage the catheter body by moving along a guide channel (e.g., a thread or track). Thus, the inner surface of the movable member may also include a keyed structure to fit into a guide or channel on the catheter body. In some variations, the movable member is threaded to fit (screw-like) into complimentary threads on the catheter body. The movable member may be moved proximally and distally along the catheter body by rotating the moveable member with respect to the catheter body. '

Fig. 2B shows a movable member that includes a screw-type thread 207 along the inner surface. The threading in the movable member mates with threading on the catheter body 215 so that the movable member may move along the catheter body by rotating the movable member with respect to the catheter body (or the catheter body with respect to the movable member). Any appropriate threading pitch and width may be used. Rotating the movable member may cause the inflatable member to twist. In some variations, the balloon catheter is configured so that the expandable member is untwisted when the balloon is expanded (e.g., when the rotatable movable member is positioned more distally), and the expandable member is twisted around the catheter body when the movable member is positioned more proximally. In some variations, the second end of the inflatable body is attached to portion of the catheter body that may rotate with respect to the rest of the catheter body, preventing twisting of the expandable member as the movable member is rotated.

The inner surface of the movable member may be configured to enhance movement with respect to the catheter body by including a lubricious surface to reduce friction between the movable member and the catheter body. The surface of the region of the catheter body adjacent to the movable member may also (or alternatively) include a friction reducing surface or material (e.g., lubricant). The movable member may also include one or more structures that enhance movement with respect to the catheter body, including bearings (e.g., ball bearings, fluid bearings, etc.). For example, bearings may be included between the catheter body and the movable member.

Fig. 2C shows a cross-section through one variation of a movable member 201 in which the movable member includes bearings 210 in recessed regions of the movable member 201. These bearings are shown as ball bearings. Bearings may be any appropriate material (e.g., metal, polymeric, etc), and may also be lubricated, or may themselves be lubricious. Bearings may be positioned anywhere around the inner surface of the movable member. In some variations, the bearings are ring-bearings encircling the catheter body.

In embodiments of the invention moveable member also forms a seal with the catheter body that substantially prevents leakage of fluid from the inflatable region. For example, the moveable member may include a gasket or lip that helps prevent substantial amounts of fluid from leaking from the inflatable region. The seal does not have to be a perfect or complete seal, and some fluid may pass from the inflatable region through the movable member. In some variations, more than one seal may be used (e.g., at both the proximal and distal ends of the movable member. In some variations, the movable member is in fluid-tight engagement with the outer surface of the catheter body.

Fig. 2D illustrates a cross-sectional view of one variation of a movable member 201 having a seal configured as an annular lip 230. The lip 230 is shown attached to the distal end of the movable member and has a region that contacts the catheter body. The lip may be made of any appropriate material, particularly flexible materials (e.g., polymeric materials) that can withstand sliding over the catheter body. For example, the lip may be made of a rubber, silicone, etc. In some variations, the seal is a gasket in some variations, the bearings described above act as a seal.

The moveable member may be configured to limit or direct the passage of fluid from the inflatable region. For example, fluid may pass into a channel within an outer delivery sheath. Delivery sheaths are described in more detail below.

Movement of the movable member may be actively or passively controlled. For example, the movable member may be moved by inflation and deflation of the inflatable member. As the inflatable member is inflated (e.g., by the addition of fluid within the inflatable region) the movable member moves distally, expanding the inflatable region radially and shortening the length of the inflatable region along the catheter body. Deflating the inflatable member (e.g., by removing the fluid from within the inflatable region) causes the movable member to move proximally, contracting the inflatable region radially and extending the length of the inflatable region along the catheter body.

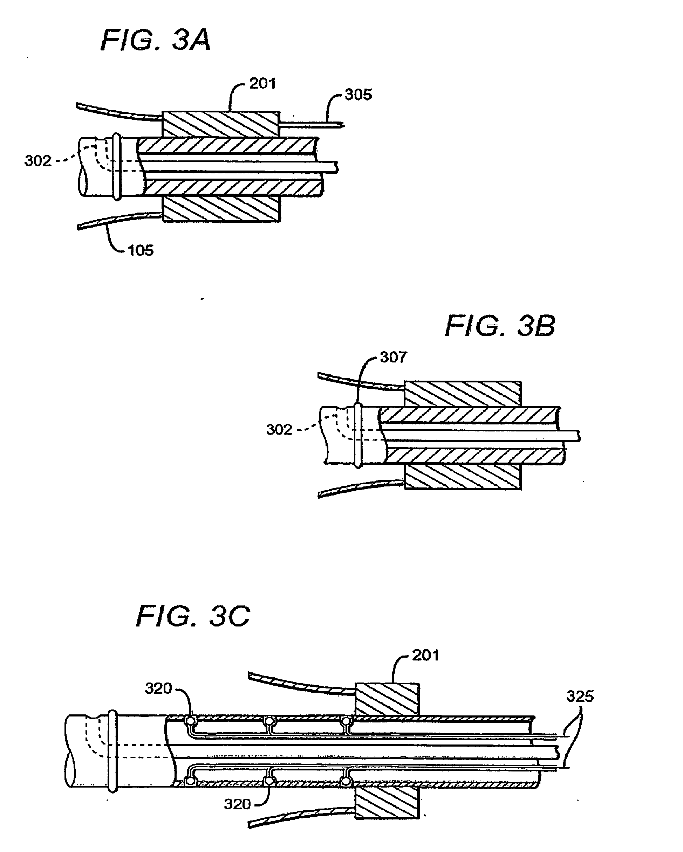

The movable member may also be coupled to a positioner that controls the position of position of the movable member (and therefore the length of the inflatable member) along the catheter body. Positioners include tubes, wires, rods, and the like. Examples of positioners are shown in Figs. 3A to 3C. The positioner may be used to move the movable member by pushing, pulling or rotating the movable member along the catheter body. The positioner may also limit the movement of the movable member along the catheter body.

Fig. 3A shows a movable member 201 connected to an inflatable member 105. The movable member is slidably disposed on a catheter body 101. The catheter body includes an inflation lumen 302 thorough which fluid may be applied/removed to inflate/deflate the inflatable region. A positioner 305 is coupled to the movable member at the proximal region of the movable member. The positioner is shown as a push wire or rod. The positioner may be used to move the movable member (e.g., to push it distally or pull it proximally), or it may be used to secure (e.g., anchor) the movable member at a selected position on the catheter body. In operation, a positioner may be used in combination with the application and/or withdrawal of fluid used to inflate/deflate the inflatable region. For example, the positioner may be used to select the axial length of the inflatable member after inflation. Thus the positioner may help determine the shape of the inflatable member. After deflation of the inflatable member, the positioner may help "retract" the inflatable member so that it is extended along the catheter body in a low-profile position. The positioner may also hold the position of the inflatable member (e.g., locking it in the low-profile position, or selecting the length of the inflated profile).

The positioner may be controlled from the proximal end of the catheter. For example, the positioner may extend proximally where it may be manipulated (e.g., manually or automatically) by a user.

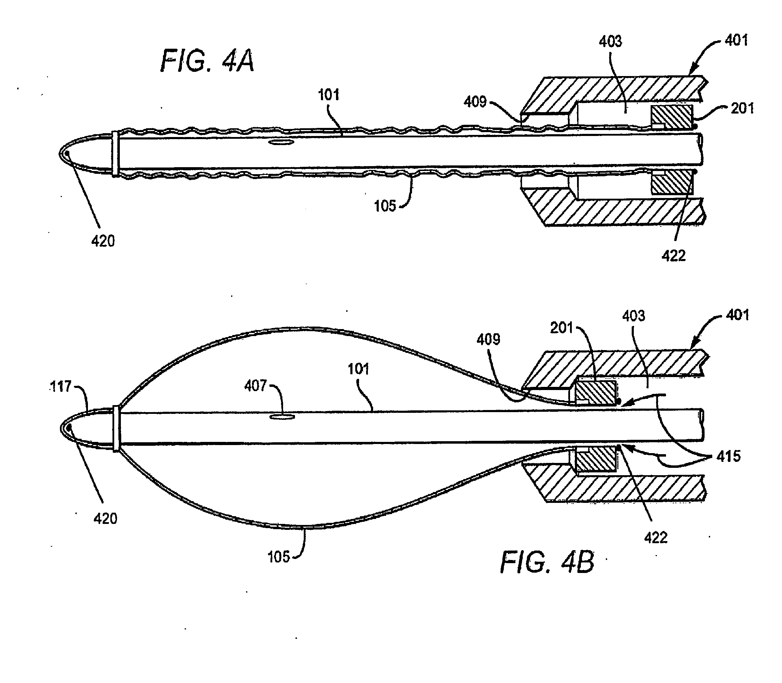

More than one positioner may be used. For example, when the positioner is configured as a push/pull wire or rod, more than one push or pull rod may be used. In some variations (as described further below in Fig. 4), the positioner may be a sheath that can couple to the movable member. The movable member may be a tube that is coaxial to the catheter body. The positioner may couple to the movable member permanently (e.g., by attachment to the movable member), as shown in Fig. 3A. In some variations, the movable member is not connected to the positioner, but the positioner holds, pushes or pulls the movable member.

The balloon catheter may also include a stop (or stops) that limit the motion of the movable member with respect to the catheter body. Fig. 3B shows one variation of a balloon catheter having a stop configured as a shoulder 307 to prevent the movable region from moving distally past the opening in the distal end wall of the catheter body that is in fluid communication with the inflation lumen 302 within the catheter body. The shoulder 307 in Fig. 3B extends radially around the catheter body, but it may be a "bump" or other structure that prevents the movable member from moving past it.

A positioner may also include one or more stops to limit or control the movement of the movable member. Fig. 3C shows one variation of a balloon catheter in which a series of stops 320 or locks are present at different positions along the length of the catheter body, and may be extended or retracted to limit the motion of the movable member either distally or proximally. In Fig. 3C, the stops can be extended or withdrawn from recesses within the catheter body to block the motion of the movable member. The stops may be extended or retracted by any appropriate means (e.g., by a pusher/pull wire, electrically, magnetically, pneumatically, etc.). In Fig. 3C the stops are shown coupled to a wire 325 or rod within the catheter shaft that can extend or withdraw them all at once. The stops may be extended or retracted *en masse,* or individually.

The balloon catheters described herein may be used with a sleeve (or delivery sheath) 401 as shown in Fig. 4A and 4B. An outer delivery sheath generally includes a lumen 403 into which the balloon catheter fits. The delivery sheath includes a distal end opening 409 through which at least a portion of the distal end of the balloon catheter passes. The outer delivery sleeve shown in Fig. 4A and 4B is also configured as a positioner that restrict the motion of the movable region 201, because it may restrict the motion of the movable member, and to position the movable member (and thereby the inflatable region) relative to the catheter body of the balloon catheter. The distal end opening 409 of the outer delivery sheath 401 is configured to permit the passage of the catheter body 101 and the inflatable member 105, but not the movable member 201. This is illustrated in Fig. 4B.

In Fig. 4B, the inflatable region (shown un-inflated in Fig. 4A) has begun to be inflated, expanding the inflatable member 105 radially from the catheter body. The inflatable region can be inflated by providing fluid from the inflation lumen within the catheter body 302, out of the opening in the distal 407 end of the wall of the catheter 407. Inflating the inflatable member results in the movement of the movable member 201 toward the distal end (shown by the arrows 415 in Fig. 4B). As the movable member moves distally, the inflatable region is permitted to expand further radially. Because the movable member cannot exit the outer delivery sheath 401, the outer delivery sheath may be positioned (e.g., axially) to control the position of the moveable member and therefore the shape of the inflatable member 105 and inflatable region.

The outer delivery sheath may also be used to limit the axial length of the inflatable region that is exposed. For example, in the balloon catheter and delivery sheath shown in Fig. 4A and 4B, the length of inflatable region exposed to the body is the length that extends distally from the opening in the outer delivery sheath.

The balloon catheters described herein may also include a distal tip 117. The distal tip may be penetrating (e.g., pointed or configured to penetrate tissue) or non-penetrating (e.g., blunted). Various designs and styles of tips may be used as part of the distal tip of the catheter body. In some variations, the second end of the inflatable member is fixed to the distal tip region. They may correspond to designs used with one or more guidewires, as are currently known. For example, the tip may include a passage for a guidewire: The port 407 can be provided with a separate lumen from the guidewire lumen. A marker may 420 be placed proximate the distal tip 117 and another marker 422 may be placed on the movable member 201 so as to delineate the axial length of the balloon. A marker material can also be incorporated into the balloon polymeric material so that a clinician can determine the size of the balloon when it is inflated. As used herein, the term "marker" indicates any type of material that allows for suitable visualization of the marker while it is inside the host. One technique is x-ray imaging of radiopaque materials such as, for example, tantalum, gold, platinum, barium sulfate, which can be in powdered form and added to the polymeric material of the balloon or inflatable member.

### Catheter Assemblies

Any of the balloon catheters described herein may be part of a catheter assembly. Catheter assemblies may include a delivery catheter in addition to a balloon catheter. The delivery catheter may be an outer delivery sheath (as described above), having a central lumen and a distal end opening. The distal end opening may be configured to allow the distal end of the balloon catheter to pass, but not allow passage of the movable member. Thus, the delivery catheter may also be configured to help position and control the balloon region of the balloon catheter. In some variations, the distal end opening is configured to allow the movable member to pass.

In operation, the catheter assembly may be used to position and engage the balloon catheter during a percutaneous (e.g., minimally invasive) procedure. For example, a guidewire or guide catheter may be inserted into a body lumen (e.g. within a subject's vasculature) and advanced into the region of the body near where the balloon catheter is to be applied (e.g., a plaque within a body vessel, etc.). The outer delivery sheath may then be positioned over the guidewire or guide lumen so that the distal end of the outer delivery sheath is near the target area. Once the outer delivery sheath is in position, the balloon catheter may be inserted into the lumen of the delivery sheath, and advanced to the distal end of the delivery sheath until at least a portion of the balloon catheter leaves the delivery sheath so that it may be inflated.

Thus, the catheter assembly may include any of the components useful for operating the balloon catheter near a target structure. For example, catheter assembly may include a guide catheter (e.g., a steerable guide catheter), a guide wire, or a combination of guide catheter and guide wire. Assemblies may also be configured as a kit, and may include any appropriate packaging (e.g., sterile or sterilizable packaging), instructions for use (e.g., written, visual, electronic, etc.), or the like.

### Methods of Using Balloon Catheters

The balloon catheters described herein may be used as part of any appropriate procedure including medical procedures such as angioplasty and stent delivery. Examples of steps that may be used as part of such methods are described herein, however it should be understood that additional steps may be used.

In general operation, a balloon catheter as described herein is introduced into an appropriate region of a body using a delivery catheter. Although the balloon catheter (particularly the low-profile balloon catheters described herein) may be configured as a delivery catheter, an additional delivery catheter (e.g., a delivery sheath) may be used. The balloon catheter may then be advanced through (or over) the delivery catheter toward a distal end of the delivery catheter. The distal end of the balloon catheter may then be passed through the distal end opening of the delivery catheter and advanced so that it is positioned adjacent to the region of the body (e.g., within the vessel) where the it is desirable to inflate the balloon. In some variations of the balloon catheter assembly described above, the distal end opening of the delivery catheter does not allow passage of the movable member of the balloon catheter.

Once the balloon catheter is positioned within the lumen, and outside of the delivery catheter, the balloon catheter may be inflated, as shown in Figs. 1B and 4B. During inflation, the movable member may be moved with respect to the outer surface of the balloon catheter body to allow the balloon to expand radially. The shape of the balloon as it is inflated may be regulated by controlling the position of the movable member. For example, the further distally that the movable member is permitted to move during inflation, the larger-diameter that the inflatable member (e.g., balloon) may expand. Thus, one or more positioners (including a delivery catheter) may be used to control the position of the movable member, and therefore the final shape of the inflation region. The movable member may be moved distally before inflation in some variations.

The balloon catheter is typically inflated by applying a fluid through the distal opening in the wall of the catheter body that is in fluid communication with a fluid (e.g., pressurized fluid) source. Any appropriate fluid may be used (including gasses and liquids), but liquids may be preferred. For example, biological saline solution (perhaps containing a biocompatible dye or contrast agent such as metrizamide, iopamidol, iothalamate sodium, iohexol, iodomide solution, or meglumine) may be used. Since fluid may be released or may "leak" in some variations, a medicament or therapeutic agent may also be used. Fluid may be applied under pressure to inflate the inflatable member. For example, fluid may be applied from within the range of about 2 atm to about 30 atm (e.g., about 5 atm to about 20 atm) through the inflatable lumen of the catheter body to exit the one or more openings in the wall of the catheter body.

Once the balloon catheter is inflated, inflation may be sustained for any desirable amount of time before the balloon catheter is deflated. The inflatable region of the balloon catheter may be deflated by allowing the pressure from within the inflatable region to force the fluid from the inflatable region. Fluid may also be actively withdrawn from the inflatable region. As the inflatable region is deflated (or after it has deflated), the movable member may be moved distally to withdrawn the inflatable member against the catheter body. The movable member may be drawn proximally by a posititoner. In some variations, the movable member moves by the action of the inflatable member as it relaxes back into the un-inflated position. In the un-inflated position, the inflatable member is collapsed coaxially against a length of the catheter body, as shown in Figs. 1A and 4A.

In one variation, the movable member and/or a region of the catheter body are threaded so that movable member moves proximally and distally by rotating around the catheter body. Thus, when the inflatable region is inflated by the addition of fluid, the movable member translates distally by rotating in a first direction (e.g., clockwise). The movable member may be withdrawn (during deflation) by rotating in the opposite direction (e.g., counterclockwise). In some variations, the inflatable member is coaxially wound around the catheter body. Inflation or deflation of the inflatable region results in winding or unwinding of the inflatable member from the catheter body. Unwinding and winding of the inflatable region by a threaded coupling between the movable member and the catheter allows for a greater selection of balloon outer diameters than typically balloon catheters (which are fixed at both ends of the inflatable member). This may be especially useful for balloon catheters having non-compliant balloons used to deal with unusual stenosis, as described above.

The balloon catheters described herein may form low-profile catheters because the inflatable member may be held adjacent to the catheter body, or even wrapped around the catheter body. Thus, the radial profile may be smaller than balloon catheters that do not allow at least one end of the balloon catheter to move.

This invention has been described and specific examples of the invention have been portrayed. While the invention has been described in terms of particular variations and illustrative figures, those of ordinary skill in the art will recognize that the invention is not limited to the variations or figures described. In addition, where methods and steps described above indicate certain events occurring in certain order, those of ordinary skill in the art will recognize that the ordering of certain steps may be modified and that such modifications are in accordance with the variations of the invention. Additionally, certain of the steps may be performed concurrently in a parallel process when possible, as well as performed sequentially as described above. Therefore, to the extent there are variations of the invention, which are within the scope of the claims, it is the intent that this patent will cover those variations as well.

## Claims

1. A balloon catheter (10), comprising:
a catheter body (101) including an inflation lumen and an opening in a distal end wall of the catheter body in fluid communication with the inflation lumen; and
an inflatable member (105) including a first end and a second end, the first end coupled to a movable member (110) that is disposed about the catheter body and forms a seal with the catheter body, the second end fixed at a distal end of the catheter body, **characterised in that**
the balloon catheter further comprises an outer delivery sheath (401) with a lumen (403), the delivery sheath having a distal end opening (404) configured to permit passage of the catheter body and inflatable member, but to prevent passage of the movable member.

2. The balloon catheter according to claim 1, wherein the catheter body includes a shoulder (307) configured to limit movement of the movable member in a distal direction.

3. The balloon catheter according to claim 1, wherein the movable member comprises an annulus including threading (207) on an inner surface configured to mate with a threading on an outer surface of the catheter body (215).

4. The balloon catheter according to claim 3, wherein the inflatable member comprises a non-compliant balloon having an inner surface area that remains essentially constant in an inflated or un-inflated state.

5. The balloon catheter according to claim 1, wherein the second end of the inflatable member is attached to a locking member permanently fixed to the catheter body.

6. The balloon catheter according to claim 1, further comprising an atraumatic tip (117) attached to the distal end of the catheter body, wherein the tip comprises a lumen configured for passage of a guidewire.

7. The balloon catheter according to claim 6, wherein the catheter body further comprises a guidewire lumen in communication with the tip lumen.

8. Balloon catheter according to claim 1, in which the inflatable member comprises a compliant balloon made from thermoplastic polymers.

9. Balloon catheter according to any one of the preceding claims, being a PTA balloon catheter.

## Patentansprüche

1. Ballonkatheter (10) mit:
einem Katheterkörper (101), der ein Aufpumplumen und eine Öffnung in einer distalen Stirnwand des Katheterkörpers aufweist, die in fluider Verbindung mit dem Aufpumplumen ist, und
ein aufpumpbares Element (105), dass ein erstes Ende und ein zweites Ende aufweist, das erste Ende mit einem beweglichen Element (110) gekoppelt, das um den Katheterkörper angeordnet ist und eine Versiegelung mit dem Katheterkörper bildet und das zweite Ende an einem distalen Ende des Katheterkörpers befestigt ist, **dadurch gekennzeichnet, dass**
der Ballonkatheter des Weiteren eine äußere Einführhülse (401) mit einem Lumen (403) aufweist, wobei die Einführhülse eine distale Endöffnung (404) aufweist, die eingerichtet ist, um einen Durchgang des Katheterkörpers und aufpumpbaren Elements zuzulassen aber einem Durchgang des beweglichen Elements vorzubeugen.

2. Ballonkatheter gemäß Anspruch 1, bei dem der Katheterkörper einenAbsatz (307) aufweist, der eingerichtet ist, die Bewegung des beweglichen Elements in einer distalen Richtung zu begrenzen.

3. Ballonkatheter gemäß Anspruch 1, bei dem das bewegliche Element auf einer Innenfläche einen Ring mit Gewinde (207) aufweist, der eingerichtet ist, sich mit einem Gewinde auf einer Außenfläche des Katheterkörpers (215) zu verbinden.

4. Ballonkatheter gemäß Anspruch 3, bei dem das aufpumpbare Element einen unnachgiebigen Ballon umfasst, der eine Innenfläche aufweist, die im Wesentlichen bei einem aufgepumpten oder unaufgepumpten Zustand gleich bleibt.

5. Ballonkatheter gemäß Anspruch 1, bei dem das zweite Ende des aufpumpbaren Elements an einem Sperrelement angebracht ist, das dauerhaft mit dem Katheterkörper verbunden ist.

6. Ballonkatheter gemäß Anspruch 1, des Weiteren mit einer atraumatischen Spitze (117), die an dem distalen Ende des Katheterkörpers angebracht ist, wobei die Spitze ein Lumen aufweist, das für den Durchgang eines Führungsdrahts eingerichtet ist.

7. Ballonkatheter gemäß Anspruch 6, bei dem der Katheterkörper des Weiteren ein Führungsdrahtlumen in Verbindung mit dem Spitzenlumen aufweist.

8. Ballonkatheter gemäß Anspruch 1, indem das aufpumpbare Element einen aus einem thermoplastischen Polymer hergestellten nachgiebigen Ballon aufweist.

9. Ballonkatheter gemäß einem der vorstehenden Ansprüche, der ein PTA-Ballonkatheter ist.

## Revendications

1. Cathéter à ballonnet (10), comprenant :
un corps de cathéter (101) incluant une lumière de gonflage et une ouverture dans une paroi d'extrémité distale du corps de cathéter en communication de fluide avec la lumière de gonflage ; et
un élément gonflable (105) incluant une première extrémité et une seconde extrémité, la première extrémité étant couplée à un élément mobile (110) qui est disposé autour du corps de cathéter et forme un joint avec le corps de cathéter, la seconde extrémité étant fixée au niveau d'une extrémité distale du corps de cathéter, **caractérisé en ce que**
le cathéter à ballonnet comprend en outre une gaine de délivrance extérieure (401) avec une lumière (403), la gaine de délivrance ayant une ouverture d'extrémité distale (404) configurée pour permettre un passage du corps de cathéter et de l'élément gonflable, mais pour empêcher un passage de l'élément mobile.

2. Cathéter à ballonnet selon la revendication 1, dans lequel le corps de cathéter inclut un épaulement (307) configuré pour limiter le mouvement de l'élément mobile dans une direction distale.

3. Cathéter à ballonnet selon la revendication 1, dans lequel l'élément mobile comprend un anneau incluant un filetage (207) sur une surface intérieure configurée pour s'accoupler avec un filetage sur une surface extérieure du corps de cathéter (215).

4. Cathéter à ballonnet selon la revendication 3, dans lequel l'élément gonflable comprend un ballonnet non flexible ayant une superficie intérieure qui reste sensiblement constante dans un état gonflé ou non gonflé.

5. Cathéter à ballonnet selon la revendication 1, dans lequel la seconde extrémité de l'élément gonflable est attachée à un élément de verrouillage fixé de manière permanente au corps de cathéter.

6. Cathéter à ballonnet selon la revendication 1, comprenant en outre une pointe non traumatisante (117) attachée à l'extrémité distale du corps de cathéter, dans lequel la pointe comprend un conduit configuré pour le passage d'un fil de guidage.

7. Cathéter à ballonnet selon la revendication 6, dans lequel le corps de cathéter comprend en outre un conduit de fil de guidage en communication avec le conduit de pointe.

8. Cathéter à ballonnet selon la revendication 1, dans lequel l'élément gonflable comprend un ballonnet flexible fait à partir de polymères thermoplastiques.

9. Cathéter à ballonnet selon l'une quelconque des revendications précédentes, étant un cathéter à ballonnet APT.
